(19) Europäisches Patentamt
European Patent Office
Office européen des brevets

(11) EP 0 343 427 B1

(12) **EUROPÄISCHE PATENTSCHRIFT**

(45) Veröffentlichungstag und Bekanntmachung des Hinweises auf die Patenterteilung:
**05.03.1997 Patentblatt 1997/10**

(51) Int. Cl.$^6$: **C07F 9/40**, C08F 30/02, C07F 9/32

(21) Anmeldenummer: **89108357.8**

(22) Anmeldetag: **10.05.1989**

(54) **Unter Verwendung von Alkenyl-phosphon- und -phosphinsäureester als Vernetzer hergestellte Hydrogele.**

Hydrogele prepared by using Alkenyl-phosphonic and phosphinic acid esters as cross-linking agents.

Hydrogels préparés en utilisant des esters d'acides alcényl-phosphoniques ou phosphiniques comme reticulants.

(84) Benannte Vertragsstaaten:
**BE CH DE ES FR GB GR IT LI NL SE**

(30) Priorität: **21.05.1988 DE 3817425**

(43) Veröffentlichungstag der Anmeldung:
**29.11.1989 Patentblatt 1989/48**

(73) Patentinhaber: **HOECHST AKTIENGESELLSCHAFT**
**65926 Frankfurt am Main (DE)**

(72) Erfinder:
- **Engelhardt, Friedrich, Dr.**
  **D-6000 Frankfurt/Main 61 (DE)**
- **Riegel, Ulrich**
  **D-6000 Frankfurt/Main 61 (DE)**
- **Gersdorf, Joachim, Dr.**
  **D-6200 Wiesbaden (DE)**
- **Kleiner, Hans-Jerg, Dr.**
  **D-6242 Kronberg (DE)**

(56) Entgegenhaltungen:
**DE-A- 2 630 693**
**FR-A- 1 375 860**
**US-A- 3 984 502**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 343 427 B1

Printed by Rank Xerox (UK) Business Services
2.13.16/3.4

## Beschreibung

Die Erfindung betrifft unter Verwendung von Alkenyl-phosphon- und -phosphinsäureestern von 1,1,1-Tris(hydroxymethyl)alkanen und von 2,2-Bis-hydroxymethyl-1,3-propandiol hergestellte Hydrogele.

Bei der Herstellung von Hydrogelen in wässriger Lösung werden als Vernetzer üblicherweise Verbindungen wie Bisacrylamidoessigsäure, Trimethylolpropantriacrylat, Tetraallyloxyethan oder ähnliche eingesetzt, wie in US-A-4 286 082 und EP-A-0 071 063 beschrieben.

Es wurde nun überraschenderweise gefunden, daß Hydrogele mit verbesserten Eigenschaften hinsichtlich Absorptionskapazität und Gelstärke erhalten werden, wenn als Vernetzer Verbindungen der allgemeinen Formel I eingesetzt werden.

Gegenstand vorliegender Erfindung sind durch Copolymerisation hydrophiler Monomerer herstellbare Hydrogele, dadurch gekennzeichnet, daß bei der Copolymerisation Verbindungen der allgemeinen Formel I

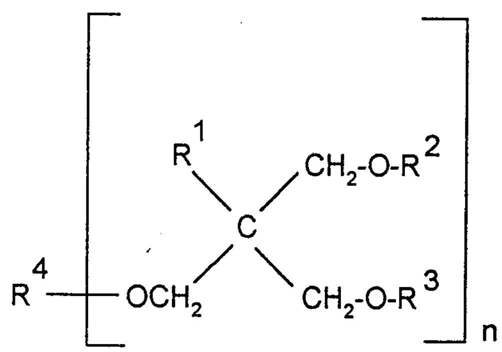

(I)

eingesetzt werden, worin

n 1 oder 2

$R^1$ Alkyl mit 1 bis 4 Kohlenstoffatomen,
$CH_2OH$ oder $CH_2OR^7$,
worin $R^7$ die Bedeutung von $R^2$ haben kann oder, falls n = 1, zusammen mit $R^4$ eine Gruppe der allgemeinen Formel II

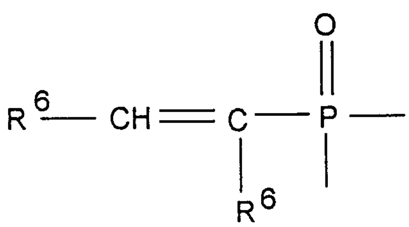

(II)

worin $R^6$ unabhängig voneinander Wasserstoff oder Alkyl mit 1 bis 4 Kohlenstoffatomen bedeutet, bilden kann,

$R^2$ eine Gruppe der allgemeinen Formel III

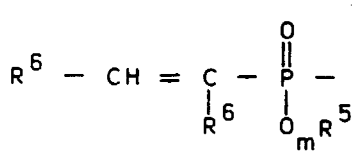

(III)

worin m 0 oder 1 und
$R^5$ alkyl mit 1 bis 4 Kohlenstoffatomen bedeutet, wobei, falls eine Verbindung der allgemeinen Formel I mehr

als eine Gruppe der allgemeinen Formel III enthält, die Reste $R^5$ unabhängig voneinander sind,

$R^3$ Wasserstoff oder eine Gruppe der allgemeinen Formel III, wobei $R^2$ und $R^3$ auch zusammen eine Gruppe der allgemeinen Formel II bilden können,

$R^4$ für n = 1

Wasserstoff, eine Gruppe der allgemeinen Formel III oder, wenn $R^1$ für Alkyl mit 1 bis 4 Kohlenstoffatomen und $R^2$ und $R^3$ zusammen für eine Gruppe der allgemeinen Formel II stehen, auch eine Gruppe der allgemeinen Formel IV

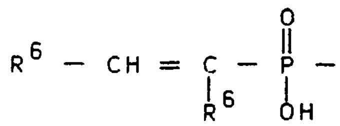

(IV)

und für n = 2

eine Gruppe der allgemeinen Formel II bedeuten.

Für $R^1$ stehendes Alkyl hat bevorzugt 1 oder 2 Kohlenstoffatome.
Für $R^5$ oder $R^6$ stehendes Alkyl hat bevorzugt 1 bis 3 Kohlenstoffatome.
$R^6$ bedeutet besonders bevorzugt Methyl und ganz besonders bevorzugt Wasserstoff.

Ganz besonders bevorzugte Verbindungen der allgemeinen Formel I sind die Verbindungen der Formel V bis XV

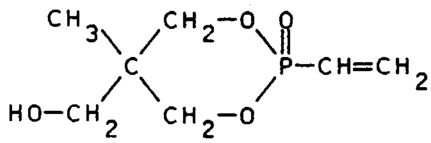

(V)

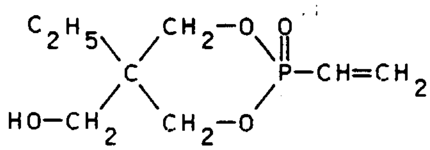

(VI)

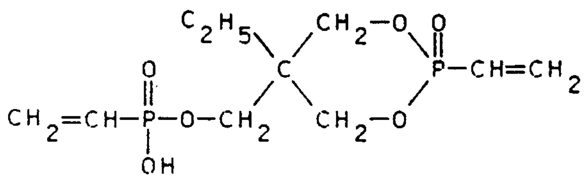

(VII)

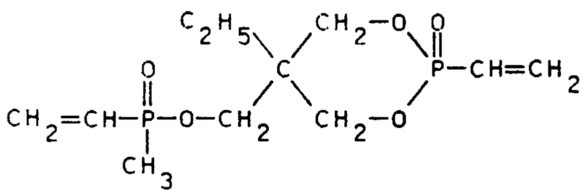

(VIII)

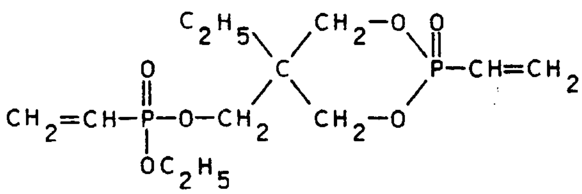

(IX)

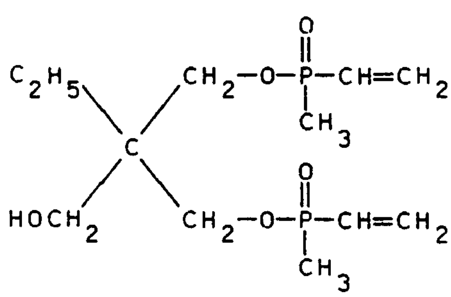

(X)

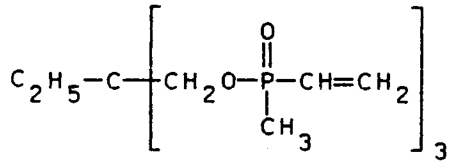

(XI)

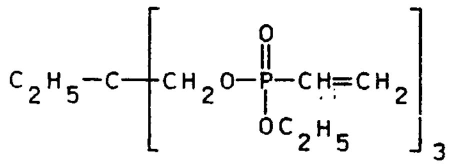

(XII)

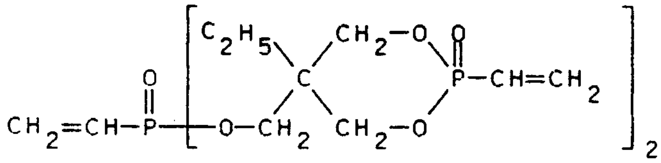

(XIII)

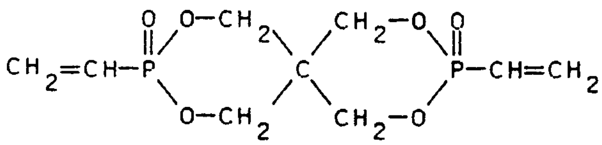

(XIV)

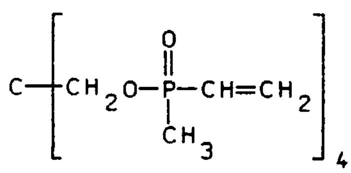

(XV)

Die Verbindungen der allgemeinen Formel I können nach verschiedenen Methoden hergestellt werden. Beispielsweise können Alkenylphosphonsäuredichloride der allgemeinen Formel XVI

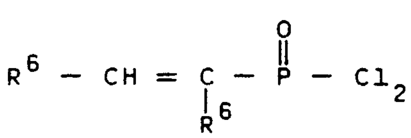

(XVI)

Alkenylphosphinsäurechloride der allgemeinen Formel XVII

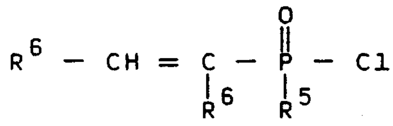 (XVII)

oder Alkenylphosphonsäureesterchloride der allgemeinen Formel XVIII

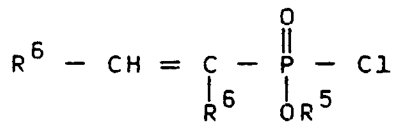 (XVIII)

wobei $R^5$ und $R^6$ die obengenannten Bedeutungen haben mit 1,1,1-(Trishydroxymethyl)alkanen, 2,2-Bis-hydroxymethyl-1,3-propandiol bzw. erfindungsgemäßen Verbindungen der allgemeinen Formel I, die noch mindestens eine freie Hydroxylgruppe enthalten, in Gegenwart von tertiären Aminen als Säurefänger in inerten Lösungsmitteln und bei geeigneten Molverhältnissen umgesetzt werden.

Geeignete tertiäre Amine sind beispielsweise Trialkylamine mit 1 bis 4 Kohlenstoffatomen pro Alkylrest, wie z.B. Triethylamin, Dialkylaniline mit 1 bis 4 Kohlenstoffatomen im Alkylrest, wie z.B. N,N'-Dimethylanilin und Pyridin.

Geeignete inerte Lösungsmittel sind beispielsweise halogenierte Kohlenwasserstoffe, wie z.B. Methylenchlorid, aromatische Kohlenwasserstoffe, wie z.B. Toluol, Ether, wie z.B. Tetrahydrofuran oder aliphatische Nitrile, wie z.B. Acetonitril.

Die Umsetzungen verlaufen vorzugsweise unter Kühlung bei -10 bis +40°C.

Die tertiären Amine werden vorzugsweise in Mengen von einem Mol pro Mol abgespaltenem HCl eingesetzt. Die Molverhältnisse Alkohol : phosphororganische Verbindung sind bevorzugt 1:1, 1:2, 1:3 oder 1:4, je nach Reaktionspartner und gewünschtem Produkt der allgemeinen Formel I.

Nach dieser Methode sind beispielsweise aus Alkenylphosphonsäuredichlorid der allgemeinen Formel XVI, 1,1,1-(Trishydroxymethyl)-alkan und tertiärem Amin im Molverhältnis 1:1:2 Verbindungen vom Typ der Formel VI, aus Alkenylphosphinsäurechlorid der allgemeinen Formel XVII, 1,1,1-(Trishydroxymethyl)-alkan und tertiärem Amin im Molverhältnis 3:1:3 Verbindungen vom Typ der Formel XI und aus Alkenylphosphonsäureesterchlorid der allgemeinen Formel XVIII, 1,1,1-(Trishydroxymethyl)-alkan und tertiärem Amin im Molverhältnis 3:1:3 Verbindungen vom Typ der Formel XII zugänglich.

Gegenbenenfalls können die so erhaltenen Verbindungen, die noch freie Hydroxylgruppen enthalten, mit Alkenylphosphonsäureanhydriden, Alkenylphosphinsäurechloriden der allgemeinen Formel XVII, Alkenylphosphonsäureesterchloriden der allgemeinen Formel XVIII oder Alkenylphosphonsäuredichloriden der allgemeinen Formel XVI zu weiteren erfindungsgemäßen Verbindungen umgesetzt werden. Diese Umsetzungen werden unter den gleichen Bedingungen wie oben beschrieben ausgeführt.

So werden beispielsweise aus einer Verbindung vom Typ der Formel VI durch Umsetzung mit einem Alkenylphosphonsäureanhydrid Verbindungen vom Typ der Formel VII, durch Umsetzung mit einem Alkenylphosphinsäurechlorid und einem tertiären Amin im Molverhältnis 1:1:1 Verbindungen vom Typ der Formel VIII, durch Umsetzung mit einem Alkenylphosphonsäureesterchlorid und einem tertiären Amin im Molverhältnis 1:1:1 Verbindungen vom Typ der Formel IX und durch Umsetzung mit einem Alkenylphosponsäuredichlorid und einem tertiären Amin im Molverhältnis 2:1:2 Verbindungen vom Typ der Formel XIII erhalten.

Verbindungen der allgemeinen Formel I können auch durch direkte Veresterung von Alkenylphosphonsäuren der allgemeinen Formel XIX

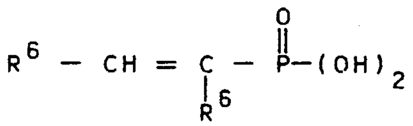 (XIX)

oder von Alkenylphosphinsäuren der allgemeinen Formel XX

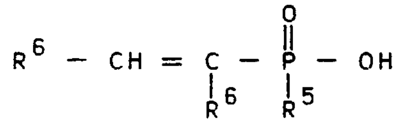

(XX)

worin $R^5$ und $R^6$ die obengenannten Bedeutungen haben mit 1,1,1-(Trishydroxymethyl)-alkanen oder 2,2-Bis-hydroxymethyl-1,3-propandiol erhalten werden.

Vorteilhafterweise werden dabei die Alkenylsäuren des Phosphors und der Alkohol im gewünschten Molverhältnis gemischt und bei Temperaturen von 150 bis 250°C, vorzugsweise bei 160 bis 220°C, unter Wasseraustritt umgesetzt. Auch hier sind die Molverhältnisse Alkohol : Säure bevorzugt 1:1, 1:2, 1:3 oder 1:4, je nach Reaktionspartner und gewünschtem Produkt der allgemeinen Formel I. Gegebenenfalls kann die Umsetzung in einem geeigneten Vakuum ausgeführt werden.

Es kann zweckmäßig sein, bestimmte bekannte Polymerisationsinhibitoren zuzusetzen wie z.B. Hydrochinon, Hydrochinonmonomethylether oder Phenothiazin. Es kann weiterhin vorteilhaft sein, die Veresterung nicht bis zur Vollständigkeit durchzuführen, sondern bei einem bestimmten Restsäuregehalt abzubrechen, da sonst die Reaktionsdauer zu lange ausgedehnt werden muß und unter diesen Bedingungen auch eine Polymerisation des Reaktionsgutes nicht immer zu verhindern ist.

Schließlich können Verbindungen der allgemeinen Formel I auch durch Umsetzung von Alkenylphosphonsäureestern der allgemeinen Formel XXI

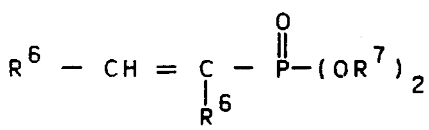

(XXI)

oder Alkenylphosphinsäureestern der allgemeinen Formel XXII

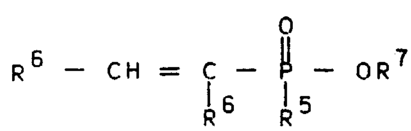

(XXII)

worin $R^5$ und $R^6$ die oben angegebenen Bedeutungen haben und $R^7$ Alkyl mit 1 bis 4 Kohlenstoffatomen bedeutet, mit 1,1,1-(Trishydroxymethyl)-alkanen oder 2,2-Bis-hydroxymethyl-1,3-propandiol mit Hilfe geeigneter Katalysatoren erhalten werden.

Geeignete Katalysatoren sind beispielsweise Alkalialkoholate oder Alkalihydride wie Natriumhydrid, ortho-Titansäureester, wie z.B. ortho-Titansäuretetraisopropylester, aber auch Alkalihydroxide, wie z.B. Kaliumhydroxid und Natriumhydroxid.

Die Umesterungsreaktionen werden bevorzugt im Vakuum im Temperaturbereich 150-250°C, bevorzugt 180-220°C, durchgeführt. Die Molverhältnisse werden bevorzugt analog zu der direkten Veresterung gewählt.

Die bei den angegebenen Verfahren anfallenden Produkte können zum Teil durch Destillation, insbesondere mit Hilfe eines Dünnschichtverdampfers, im Hochvakuum gereinigt werden. Teilweise können sie aber auch direkt als Rohprodukt weiterverarbeitet werden.

2,2-Bis-hydroxymethyl-1,3-propandiol ist eine käufliche Verbindung. 1,1,1-(Trishydroxymethyl)-alkane haben bevorzugt 5 bis 8, besonders bevorzugt 5 oder 6 Kohlenstoffatome. Sie sind ebenfalls käuflich oder können nach bekannten Methoden hergestellt werden (z.B. Houben-Weyl, Methoden der Organischen Chemie, Georg Thieme Verlag, Stuttgart, Bd.6/1a/2, 1980, Seite 1314).

Ebenso sind die als Ausgangsstoff für die Synthese der Verbindungen der allgemeinen Formel I genannten phosphororganischen Verbindungen der allgemeinen Formeln XVI bis XXII käuflich oder nach gängigen Methoden zugänglich (z.B. Houben-Weyl, Methoden der Organischen Chemie, Georg Thieme Verlag, Stuttgart, Bd. 12/1, 1963, Seiten 217 ff und 338 ff).

Die Verbindungen der allgemeinen Formel I, die mindestens zwei Doppelbindungen enthalten, finden Verwendung

als sogenannte Vernetzer, das heißt als polymerisierbare, mehrfach ungesättigte Monomere, bei der Synthese von Polymeren, insbesondere von wasserquellbaren Hydrogelen aus ungesättigten Monomeren. Ein wesentlicher Vorteil ist dabei deren sehr gute Löslichkeit sowohl in polaren als auch in unpolaren Lösungsmitteln. So sind die Verbindungen der allgemeinen Formel I im Gegensatz zu zum Beispiel Trimethylolpropantriacrylat oder Trimethylolpropantrimethacrylat in jedem Verhältnis mit Wasser mischbar. Es handelt sich darüber hinaus um schwerflüchtige, geruchsarme Substanzen. Daraus resultiert als weiterer Vorteil eine wesentlich verringerte Neigung zur Diffusion oder Verdunstung während des Polymerisationsprozesses.

Verbindungen der allgemeinen Formel I, die noch mindestens eine freie Hydroxylgruppe enthalten, können auch als Ausgangsverbindung zur Synthese weiterer Verbindungen verwendet werden.

Als hydrophile Monomere kommen insbesondere Acrylsäure, Methacrylsäure, Crotonsäure, 2-Acrylamido-2-methylpropansulfonsäure und -phosphonsäure, Vinylphosphonsäure, Vinylphosphonsäurehalbester, deren Salze, Acrylamid, N-Vinylamide oder Gemische davon in Frage. Bevorzugt ist Acrylsäure und deren Salze.

Die Verbindungen der allgemeinen Formel I werden vorzugsweise in Mengen von 0,05 bis 20 Gew. %, bezogen auf das Gesamtmonomerengewicht, eingesetzt.

Die Polymerisation kann in homogener Phase z.B. in wässriger Lösung als sog. Gelpolymerisation oder nach dem Verfahren der Inversemulsionspolymerisation durchgeführt werden. Eine weitere Möglichkeit zur Synthese der erfindungsgemäßen Hydrogele bietet die Fällungspolymerisation aus organischen Lösungsmitteln, wie zum Beispiel Alkoholen, bevorzugt tert. Butanol, oder Kohlenwasserstoffen wie Hexan oder Cyclohexan.

Die Polymerisation kann durch Radikalbildner wie zum Beispiel organische oder anorganische Peroxide sowie Azoverbindungen ausgelöst werden. Beispiele sind Benzoylperoxid, tert. Butylhydroperoxid, Cumolhydroperoxid, $(NH_4)_2S_2O_8$, $K_2S_2O_8$, $H_2O_2$ oder Azo-diisobutyronitril. Auch Redoxsysteme eignen sich in hervorragender Weise als Polymerisationsinitiatoren.

Die Polymerisation kann schließlich auch durch energiereiche Strahlung ausgelöst werden.

Die erfindungsgemäßen Hydrogele eignen sich in hervorragender Weise als Absorbentien für wässrige Flüssigkeiten, zur Formulierung kosmetischer Zubereitungen, sowie als Additive in Bohrspülungen und Zementschlämmen bei der Erdölgewinnung.

Besonders vorteilhaft verhalten sich Copolymere aus Acrylsäure und den Verbindungen der allgemeinen Formel I als sogenannte super absorbing polymers (SAP) beim Einsatz in Hygieneartikeln, wie z.B. Windeln, wobei die Acrylsäure teilweise als Alkali- oder Ammoniumsalz vorliegen kann. Die Neutralisation kann sowohl vor als auch nach der Polymerisation erfolgen.

Die erfindungsgemäßen wasserquellbaren Hydrogele zeichnen sich gegenüber den Verbindungen des Standes der Technik durch eine homogenere Netzwerkstruktur aus, da die Verbindungen der allgemeinen Formel I sowohl in Wasser als auch in polaren organischen Lösungsmitteln völlig löslich sind. Dadurch weisen die Hydrogele hohe Absorptionskapazitäten neben hoher Gelstärke auf.

Die folgenden Beispiele 1 bis 12 erläutern die Herstellung der Verbindungen der allgemeinen Formel I. Die Beispiele 13 bis 34 erläutern die vorliegende Erfindung bezüglich der Herstellung der Hydrogele.

Beispiel 1

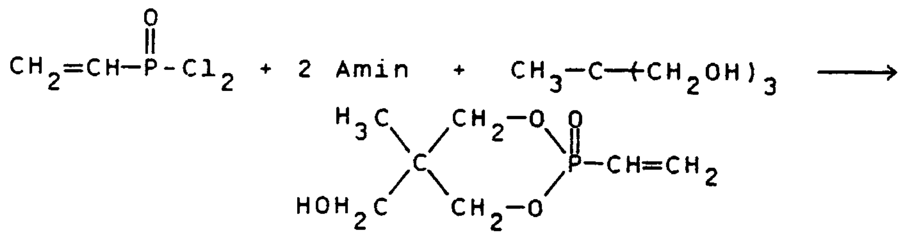

(V)

240 g (2,0 mol) 1,1,1-(Trishydroxymethyl)ethan werden zusammen mit 404 g (4,0 mol) Triethylamin in 1600 ml Tetrahydrofuran gegeben. Unter lebhaftem Rühren werden 290 g (2,0 mol) Vinylphosphonsäuredichlorid während 2 Stunden unter Kühlung bei 20°C eingetropft. Dann wird 15 Stunden nachgerührt und anschließend das gebildete Triethylaminhydrochlorid abgesaugt. Es wird mit Tetrahydrofuran gewaschen und das Filtrat durch Destillation im Vakuum von Tetrahydrofuran befreit. Der Rückstand wird über einen Dünnschichtverdampfer bei 0,067 kPa und einer Badtemperatur von 240°C destilliert. Man erhält 345 g, Fp.: 70-75°C. Durch eine Destillationsversuch wird der Siedepunkt bestimmt: 213-215°C/0,053 kPa. Das Produkt fällt als Diastereomerengemisch an. Die Ausbeute beträgt 90% d.Th.

| $C_7H_{13}O_4P$ (192) | | | | | | |
|---|---|---|---|---|---|---|
| ber.: | 43,75% | C | 6,77% | H | 16,15% | P |
| gef.: | 43,6% | C | 6,8% | H | 16,1% | P |

Beispiel 2

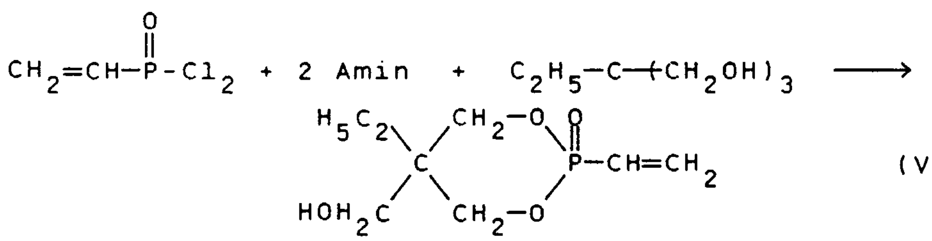

(VI)

280 g (2,09 mol) 1,1,1-(Trishydroxymethyl)propan und 422 g (4,18 mol) Triethylamin werden in 1600 ml Tetrahydrofuran gelöst. Dann werden unter lebhaftem Rühren 303 g (2,09 mol) Vinylphosphonsäuredichlorid während 3 Stunden unter Kühlung bei 20°C eingetropft. 15 Stunden wird nachgerührt und anschließend das gebildete Triethylaminhydrochlorid abgesaugt. Es wird mit Tetrahydrofuran nachgewaschen und das Filtrat durch Destillation im Vakuum von Tetrahydrofuran befreit. Der Rückstand wird über einen Dünnschichtverdampfer bei 0,027 kPa und einer Badtemperatur von 240°C destilliert. Man erhält 370 g, das Produkt hat einen Erstarrungspunkt von ca. 25°C. Durch einen Destillationsversuch wird der Siedepunkt bestimmt: 199°C/0,013 kPa. $n_D^{20}$ : 1,4890. Das Produkt fällt als Diasteromerengemisch an. Die Ausbeute beträgt 86% d.Th.

| $C_8H_{15}O_4P$ (206) | | | | | | |
|---|---|---|---|---|---|---|
| ber.: | 46,60% | C | 7,28% | H | 15,05% | P |
| gef.: | 46,4% | C | 7,3% | H | 14,8% | P |

Beispiel 3

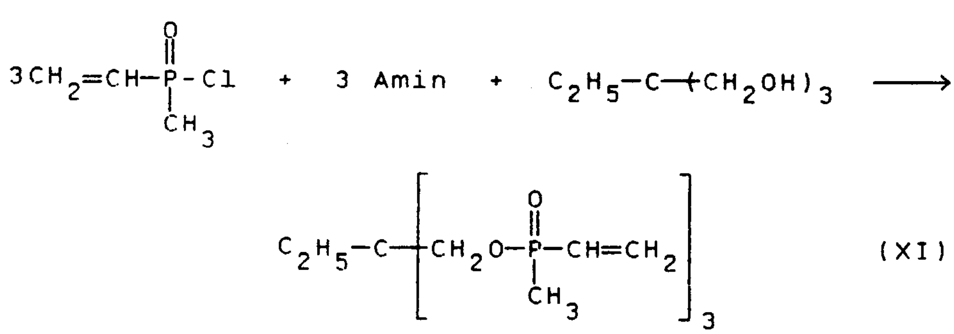

(XI)

20 g (0,15 mol) 1,1,1-(Trishydroxymethyl)propan und 45,5 g (0,45 mol) Triethylamin werden in 150 ml Toluol gegeben. Unter lebhaftem Rühren werden 56 g (0,45 mol) Methylvinylphosphinsäurechlorid unter Kühlung bei 20°C eingetropft. Dann wird 15 Stunden nachgerührt und anschließend das gebildete Triethylaminhydrochlorid abgesaugt. Es wird mit Toluol nachgewaschen und das Filtrat durch Destillation im Vakuum vom Toluol befreit. Es verbleiben 58 g des Produktes mit einem Brechungsindex $n_D^{20}$ : 1,4942. Das Produkt läßt sich über einen Dünnschichtverdampfer bie 0,027 kPa und einer Badtemperatur von 260-270°C destilliert. Die Ausbeute an dem Rohprodukt beträgt 97% d.Th.

| $C_{15}H_{29}O_6P_3$ (398) | | | | | |
|---|---|---|---|---|---|
| ber.: | 23,37% | P | gef.: | 23,1% | P |

Beispiel 4

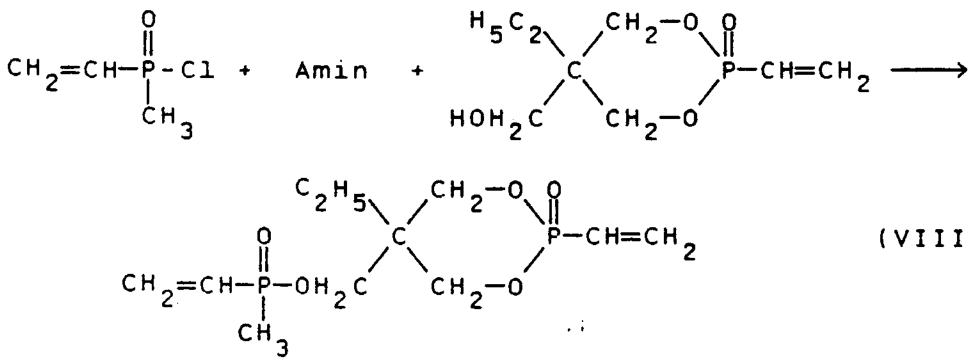

(VIII)

76 g (0,34 mol) der Verbindung aus Beispiel 2 werden zusammen mit 34,4 g (0,34 mol) Triethylamin in 100 ml Tetrahydrofuran gegeben. Nun werden 42,5 g (0,34 mol) Methylvinylphosphinsäurechlorid unter lebhaftem Rühren unter Kühlung bei 20°C eingetropft. Dann wird 15 Stunden nachgerührt und anschließend das gebildete Triethylaminhydrochlorid abgesaugt. Es wird mit Tetrahydrofuran gewaschen und das Filtrat durch Destillation im Vakuum von Tetrahydrofuran befreit. Es verbleiben 95 g der Verbindung VIII. Durch eine Destillationsversuch wird der Siedepunkt bestimmt: 205-210°C/0,067 kPa. Die Ausbeute an dem Rohprodukt beträgt 95% d.Th.

| $C_{11}H_{20}O_5P_2$ (294) | | | | | |
|---|---|---|---|---|---|
| ber.: | 21,09% | P | gef.: | 20,5% | P |

Beispiel 5

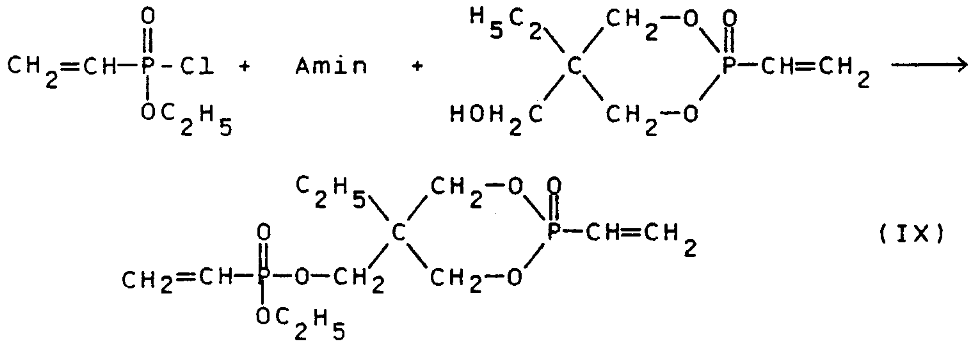

(IX)

34,7g (0,168 mol) der Verbindung aus Beispiel 2 und 17 g (0,168 mol) Triethylamin werden in 100 ml Toluol gegeben. Dann werden 26 g (0,168 mol) Vinylphosphonsäureethylesterchlorid unter Rühren und Kühlen bei 20°C eingetropft. Es wird nachgerührt und abgesaugt. Das Filtrat wird nach dem Nachspülen mit Toluol von dem Lösungsmittel im Vakuum befreit. Es verbleiben 52,5 g der Verbindung IX, $n_D^{20}$ : 1,4848 Das Produkt kann im Dünnschichtverdampfer bei 0,027 kPa und einer Badtemperatur von 240°C destilliert werden. Die Ausbeute an dem Rohprodukt beträgt 96% d.Th.

| $C_{12}H_{22}O_6P_2$ (324) | | | | | |
|---|---|---|---|---|---|
| ber.: | 19,13% | P | gef.: | 18,7% | P |

Beispiel 6

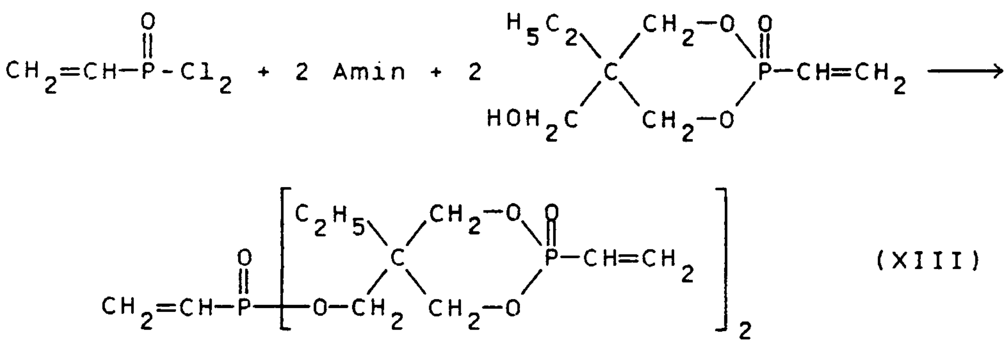

(XIII)

81,4g (0,4 mol) der Verbindung aus Beispiel 2 und 40,4 g (0,4 mol) Triethylamin werden in 100 ml Toluol gegeben. Dann werden 29 g (0,2 mol) Vinylphosphonsäuredichlorid unter Rühren und Kühlen bei 20°C eingetropft. Es wird nachgerührt, abgesaugt und mit Toluol nachgewaschen. Das Filtrat wird im Vakuum vom Toluol befreit. Es verbleiben 97 g des Produkts mit dem Brechungsindex $n_D^{20}$ : 1,4945. Das entspricht einer Ausbeute von 100% d.Th.

| $C_{18}H_{31}O_9P_3$ (484) | | | | | |
|---|---|---|---|---|---|
| ber.: | 19,21% | P | gef.: | 19,2% | P |

Beispiel 7

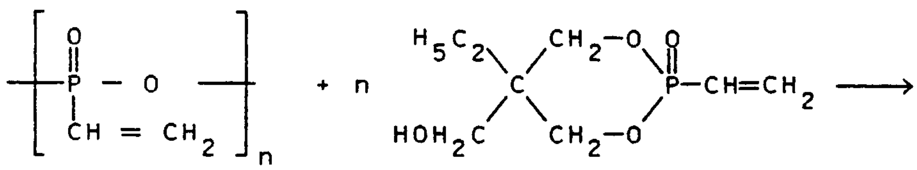

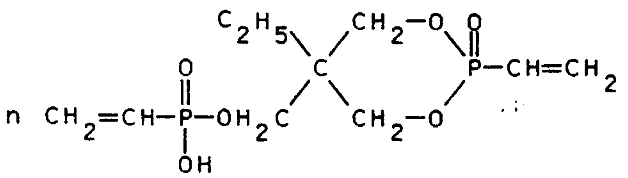

(VII)

321,4g (1,56 mol) der Verbindung aus Beispiel 2 werden in 321,4 g Methylenchlorid gelöst. Dann werden 280,8 g (0,2 mol) einer 50%igen Lösung des Vinylphosphonsäure-anhydrids in Methylenchlorid innerhalb einer Stunde unter Rühren eingetropft. Dabei steigt die Temperatur auf 33°C und bleibt bei dieser Temperatur 30 min. Dann wird 3 Stunden nachgerührt und anschließend noch 4 Stunden am Rückfluß gekocht. Anschließend wird im Vakuum das Lösungsmittel abdestilliert während ca. 3 Stunden bis zu einer Innentemperatur von 50°C. Es verbleiben 130 g eines Rohproduktes. Das Rohprodukt hat den Brechungsindex $n_D^{20}$ : 1,4945. Aufgrund eines $^{31}$P-NMR Spektrums handelt es sich um ein Gemisch zweier Diastereomerer, das in dem Rohprodukt zu etwa 60% vorliegt ($d_6$-DMSO; $\delta$ = 14,79; 14,82; 11,78; 12,73 ppm). Das Ausgangsprodukt, die Verbindung aus Beispiel 2 liegt zu 8% Vinylphosphonsäure zu 9% und Vinylpyrophosphonsäure zu 4% vor.
$C_{10}H_{18}O_6P_2$ (296)

Beispiel 8

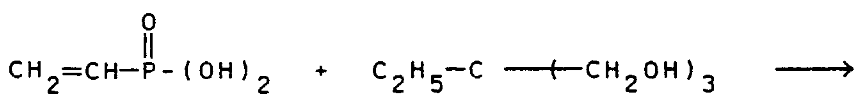

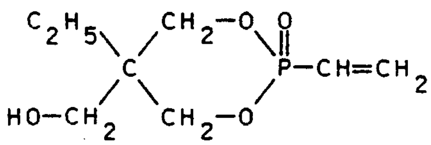

(VI)

53,7 g (0,4 mol) 1,1,1-(Trishydroxymethyl)propan und 43,2 g (0,4 mol) Vinylphosphonsäure werden unter Rühren bei 0,067 bis 0,133 kPa auf 160-170°C erwärmt. Bei abfallendem Vakuum sammelt sich in einer der Reaktionsapparatur nachgeschalteten Kühlfalle Wasser. Wenn sich etwa 7,5 g Wasser gesammelt haben, wird die Reaktionstemperatur bei wieder verbessertem Vakuum bis auf 200°C gesteigert und eine Stunde bei dieser Temperatur gehalten. Das angefallene Reaktionsgut hat nun eine Säurezahl von 134 mg KOH/g Substanz. Anschließend wird im Dünnschichtverdampfer bei 0,067 bis 0,133 kPa und einer Badtemperatur von 270°C destilliert. Das anfallende Produkt hat eine Säurezahl von 38 mg KOH/g Substanz und einen Gehalt von 76% aufgrund des $^{31}$P-NMR Spektrums.

Beispiel 9

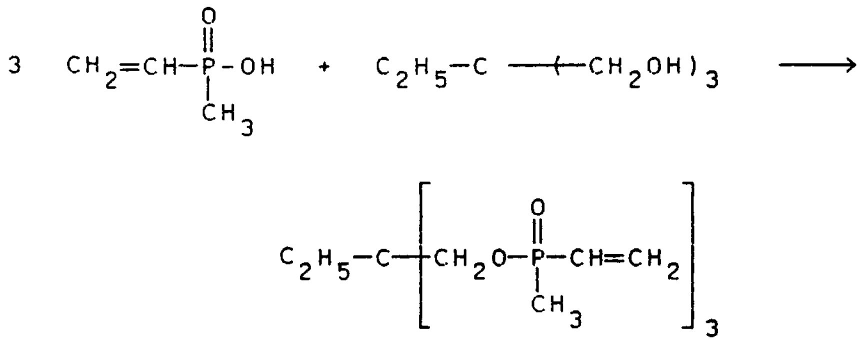

(XI)

33,6 g (0,25 mol) 1,1,1-(Trishydroxymethyl)propan und 79,5 g (0,75 mol) Methyl-vinylphosphinsäure werden bei 0,067 bis 0,133 kPa unter lebhaftem Rühren auf 150°C und dann schrittweise während mehrerer Stunden bis auf 190-195°C erhitzt. In einer der Reaktionsapparatur nachgeschalteten Kühlfalle sammeln sich 12 g Wasser. Das angefallene Reaktionsgut hat eine Säurezahl von 134 mg KOH/g Substanz. Bei 260-270°C und 0,133 kPa wird das Produkt in einem Dünnschichtverdampfer destilliert. Man erhält 81 g mit einem Gehalt von ca. 70% aufgrund des $^{31}$P-NMR Spektrums.

Beispiel 10

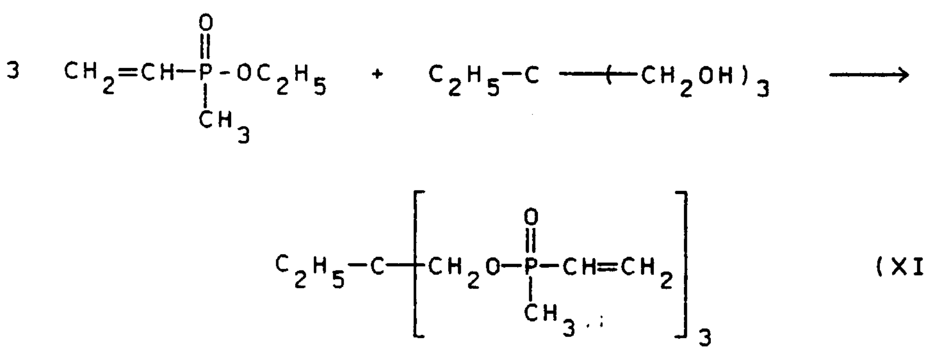

(XI)

23,5 g (0,175 mol) 1,1,1-(Trishydroxymethyl)propan, 70,4 g (0,525 mol) Methyl-vinylphosphinsäureethylester und 2,3 g ortho-Titansäuretetra-iso-propylester werden auf 150°C und dann schrittweise im Verlauf mehrerer Stunden unter lebhaftem Rühren bis 210°C erhitzt. Insgesamt werden 12 g Ethanol abdestilliert. Das angefallene Reaktionsgut hat eine Säurezahl von 75 mg KOH/g Substanz. Es kann bei 0,053 kPa und einer Badtemperatur von 250-260°C in einem Dünnschichtverdampfer destilliert werden.

Beispiel 11

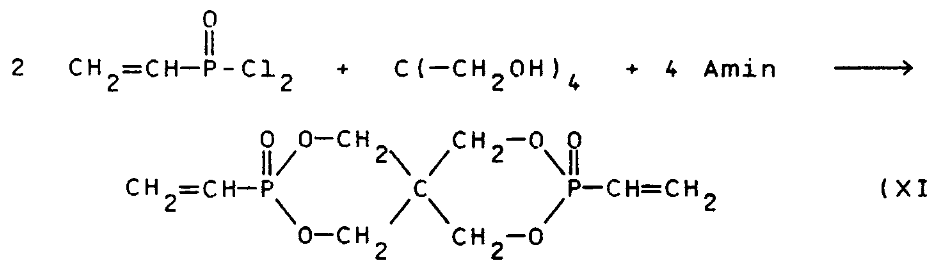

(XIV)

68,1 g (0,5 mol) 2,2-Bis-(hydroxymethyl)-1,3-propandiol werden in 400 ml Acetonitril gegeben, dazu werden 202,4 g (2,0 mol) Triethylamin unter Rühren zugetropft. Dann werden 145 g (1 mol) Vinylphosphonsäuredichlorid bei 30°C zugetropft und anschließend 15 Stunden nachgerührt. Dann wird zum Rückfluß erhitzt und nach ca. 15 Minuten heiß abgesaugt. Aus dem Filtrat fallen erneut Kristalle aus, die ebenfalls abgesaugt werden, insgesamt erhält man ca. 265 g Triethylaminhydrochlorid. Dann wird das Acetonitril aus dem Filtrat abdestilliert und der Rückstand mit Aceton digeriert. Man erhält 125 g Rohprodukt. Dieses wird aus Isopropanol umkristallisiert, Fp.: 161°C. Die Ausbeute an dem Rohprodukt beträgt ca. 90% d.Th.

| $C_9H_{14}O_8P_2$ (280) | | | | | | |
|---|---|---|---|---|---|---|
| ber.: | 38,57% | C | 5,0% | H | 22,14% | P |
| gef.: | 38,3% | C | 4,8% | H | 21,0% | P |

Beispiel 12

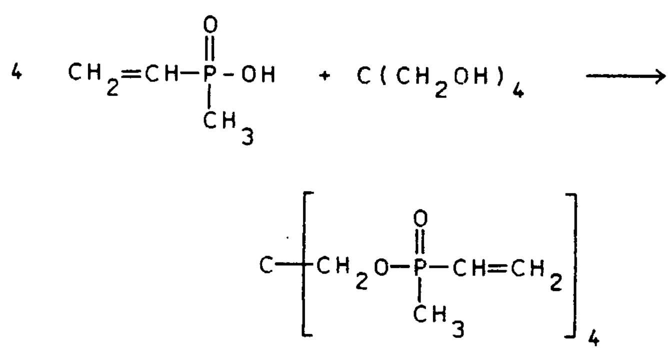

(XV)

13,6 g (0,1 mol) 2,2-Bis-(Hydroxymethyl)-1,3-propandiol und 42,4 g (0,4 mol) Methyl-vinylphosphinsäure werden unter lebhaftem Rühren schrittweise während etwa 5 Stunden auf 155 bis 190°C bei etwa 0,067 kPa erhitzt. In einer der Reaktionsapparatur nachgeschalteten Kühlfalle sammeln sich etwa 6 g Wasser. Das anfallende Rohprodukt hat eine Säurezahl von 115 mg KOH/g Substanz. Der Brechungsindex beträgt $n_D^{20}$ : 1,4911.

| $C_{78}H_{32}O_8P_4$ (488) | | | | | |
|---|---|---|---|---|---|
| ber.: | 25,41% | P | gef.: | 24,2% | P |

Beispiel 13

In einem durch geschäumtes Kunststoffmaterial gut isolierten Polyethylengefäß mit einem Fassungsvermögen von 10 l werden 5500 g E-Wasser vorgelegt, 1740 g Natriumbicarbonat darin dispergiert und langsam 1985 g Acrylsäure so zudosiert, daß ein Überschäumen der Reaktionslösung vermieden wird, wobei sich diese auf eine Temperatur von ca. 10 - 8°C abkühlt. Es werden nun 15 g der Verbindung VII, hergestellt gemäß Beispiel 7, und 10 g eines Natrium-Diisooctylsulfosuccinates (Rewopol V 2133 der Firma REWO, Steinau) zugegeben. Bei einer Temperatur von 1-10°C werden die Initiatoren, ein Redoxsystem, bestehend aus 6 g Kaliumperoxidisulfat, gelöst in 170 g Wasser und 0,2 g Ascorbinsäure, gelöst in 20 g Wasser, nacheinander zugegeben und gut verrührt. Die Reaktionslösung wird daraufhin ohne Rühren stehen gelassen, wobei durch einsetzende Polymerisation, in dessen Verlauf die Temperatur bis auf ca. 85°C ansteigt, ein festes Gel entsteht. Dieses wird anschließend mechanisch zerkleinert, bei Temperaturen über 80° getrocknet und gemahlen.

Das vorstehend beschriebene Produkt wurde in herkömmlicher Weise in eine Babywindel eingearbeitet und zeichnete sich hier durch eine besonders gute Flüssigkeitsretention aus.

Beispiel 14

In einem 10 Liter-Kunststoffgefäß werden 4290 g Eis und 1970 g Acrylsäure vorgelegt und langsam 1655 g NaOH 50%ig zudosiert, anschließend 30 g der Verbindung VII, hergestellt gemäß Beispiel 7, gelöst in 100 g Wasser und 10 g Rewopol V 2133 zugegeben. Die Reaktionslösung wird auf 20°C eingestellt und anschließend mit den Initiatoren, ein Redoxsystem bestehend aus 6 g Kaliumperoxidisulfat, gelöst in 170 g Wasser, und 0,2 g Ascorbinsäure, gelöst in 120 g Wasser, versetzt und ohne Rühren stehen gelassen. Das durch Polymerisation entstehende Gel wird anschließend mechanisch zerkleinert, bei Temperaturen über 80°C getrocknet und gemahlen.

Beispiel 15

In einem 10-Liter-Polyethylengefäß werden 5250 g E-Wasser, 1988 g Acrylsäure und 12 g der Verbindung XIII, hergestellt gemäß Beispiel 6, vorgelegt und 10 g Rewopol V 2133 eingerührt. Nach Einstellen der Reaktionslösung auf 18-20°C werden die Initiatoren, 6 g Kaliumperoxidisulfat in 170 g Wasser und 0,2 g Ascorbinsäure in 20 g Wasser nacheinander zugegeben und das Reaktionsgemisch gut isoliert ohne Rühren stehen gelassen. Nach einsetzender Reaktion steigt die Temperatur bis auf ca. 90°C an, und es entsteht ein festes Gel. Dieses wird mechanisch durch einen Extruder zerkleinert, dem kontinuierlich 1555 g NaOH 50%ig zudosiert werden, wobei teilweise Verdampfung des Wassers erfolgt. Das flockige Polymer wird anschließend bei Temperturen über 80°C endgetrocknet und gemahlen.

Beispiel 16

In einen durch geschäumtes Kunststoffmaterial gut isolierten Polyethylengefäß mit einem Fassungsvermögen von 10 l werden 5380 g E-Wasser vorgelegt, 1740 g Natriumbicarbonat darin dispergiert und langsam 1985 g Acrylsäure so zudosiert, daß ein Überschäumen der Reaktionslösung vermieden wird, wobei sich diese auf eine Temperatur von ca. 10 - 8°C abkühlt. Es werden nun 15 g der Verbindung XI, hergestellt gemäß Beispiel 3, und 10 g eines Natrium-Diisooctylsulfosuccinates (Rewopol V 2133 der Firma REWO, Steinau) zugegeben. Bei einer Tempratur von 6-8°C werden die Initiatoren, ein Redoxsystem, bestehend aus 2,2 g 2,2′-Azobisamidinopropan-Dihydrochlorid, gelöst in 20 g Wasser und 0,2 g Ascorbinsäure, gelöst in 20 g Wasser, 4,4 g Kaliumperoxidisulfat, gelöst in 170 g Wasser, und 6 g Natriumpyrosulfit, gelöst in 120 g Wasser, nacheinander zugegeben und gut verrührt. Die Reaktionslösung wird daraufhin ohne Rühren stehen gelassen, wobei durch einsetzende Polymerisation, in dessen Veralauf die Temperatur bis auf ca. 85°C ansteigt, ein festes Gel entsteht. Dieses wird anschließend mechanisch zerkleinert, bei Temperaturen über 80° getrocknet und gemahlen.

Das vorstehend beschriebene Produkt wurde in herkömmlicher Weise in eine Babywindel eingearbeitet und zeichnete sich hier durch eine besonders gute Flüssigkeitsretention aus.

Beispiel 17

In einem 1 Liter-Polymerisationskolben aus Glas, versehen mit Rührwerk, Thermometer, Rückflußkühler, Gaseinleitungsrohr und Wasserbad werden 468 g tert. Butanol (600 ml) vorgelegt und 0,1 g der Verbindung V, hergestellt gemäß Beispiel 1, und 65 g APS unter Rühren darin suspendiert. Über das Gaseinleitungsrohr werden nun 5,5 g Ammoniak-Gas eingeleitet, wobei eine schwach trübe Lösung entsteht. Der pH dieser Lösung muß >7 betragen. Anschließend werden 15 g Acrylamid und 20 g N-Vinyl-N-methylacetamid zugegeben und die Lösung unter Einleiten eines schwachen Stickstoff-Stromes auf eine Temperatur von 50°C angeheizt. Es wird jetzt 1,0 g POROFOR N zugegeben. Die Rührgeschwindigkeit wird auf 60-80U/Min gedrosselt, das Einleiten des Stickstoffstromes wird beibehalten.

Nach ca. 20 min setzt die Polymerisation ein, was an einem Ausflocken des Polymerisates sowie einem geringen Temperaturanstieg festzustellen ist. Im Verlauf von ca. 1 Std. entsteht ein dicker, gerade noch rührbarer Brei, und die Temperatur steigt bis max. 70°C an. Nach Erreichen des Temperaturmaximums wird 2 Stunden bei 80°C Badtemperatur nachgerührt. Der Rückflußkühler wird jetzt gegen eine Destillationsbrücke ausgetauscht, und das tert. Butanol wird nach Abkühlung auf 60°C unter Wasserstrahlvakuum abdestilliert. Als fertiges Produkt erhält man 105 g weißes Pulver mit einem Schüttgewicht von ca. 0,2 kg/l, das sich hervorragend als Additiv in Bohrspülungen und Zementschlämmen bei der Erdölgewinnung eignet.

Beispiel 18

In einem 1 Liter-Polymerisationskolben aus Glas, versehen mit Rührwerk, Thermometer und Rückflußkühler werden 600 ml Hexan vorgelegt und 98,9 g Acrylsäure sowie 1,1 g der Verbindung XIII, hergestellt gemäß Beispiel 6, darin gelöst. Unter Einleiten eines schwachen $N_2$-Stromes wird mittels eines elektrisch beheizten Wasserbades auf 68°C angeheizt, worauf die Zugabe von 1,0 g Di-Laurylperoxid erfolgt. Nach Einsetzen der Polymerisation tritt deutlicher Rückfluß ein, und das entstandene Polymer flockt aus. Es wird 3 Stunden unter Rückfluß nachgerührt, dann das Polymer abgesaugt und im Trockenschrank bis zur Gewichtskonstanz getrocknet. Man erhält 100 g eines weißen Pulvers, das als saurer Verdicker in kosmetischen Zubereitungen eingesetzt werden kann.

Weitere Beispiele zur Herstellung erfindungsgemäßer Polymerisate gemäß der hier beschriebenen Beispiele 13 bis 18 sind in folgender Tabelle 1 zusammengefaßt. Die Mengenangaben bedeuten Gewichsprozente, bezogen auf Gesamtmonomeranteil.

Folgende Abkürzungen werden benutzt:

AS: Acrylsäure
MAS: Methacrylsäure
CTS: Crotonsäure
VPS: Vinylphosphonsäure
VPE: Vinylphosphonsäurehalbester
APS: 2-Acrylamido-2-methyl-propansulfonsäure
AMPP: 2-Acrylamido-2-methyl-propanphosphonsäure
AM: Acrylamid
VIMA: N-Vinyl-N-methylacetamid

Tabelle 1

| Beisp. | hergest. analog Beispiel | AS % | MAS % | APS % | AMPP % | VPS % | VPE % | CTS % | AM % | VIMA % | erfindungsgemäße Verbindung Formel | gemäß Beisp. | % | Neutralisationsgrad |
|---|---|---|---|---|---|---|---|---|---|---|---|---|---|---|
| 19 | 16 | 79,25 | | 20,00 | | | | | | | 4 | 4 | 0,75 | 75 |
| 20 | 14 | 98,50 | | | | | | | | | 10 | 11 | 1,50 | 75 |
| 21 | 16 | 99,50 | | | | | | | | | 11 | 12 | 0,50 | 50 |
| 22 | 15 | 99,25 | | | | | | | | | 3 | 7 | 0,75 | 65 |
| 23 | 13 | 99,25 | | | | | | | | | 5 | 5 | 0,75 | 65 |
| 24 | 16 | 80,0 | 10,0 | 9,5 | | | | | | | 6 | | 0,5 | 48 |
| 25 | 16 | 70,0 | | 25,0 | | 4,0 | | | | | 3 | 7 | 1,0 | 75 |
| 26 | 16 | 75,0 | | 20,0 | | | 4,0 | | | | 3 | 7 | 1,0 | 75 |
| 27 | 16 | 85,0 | 10,0 | | | | 4,0 | | | | 7 | 3 | 1,0 | 70 |
| 28 | 16 | 40,0 | | 25,0 | 4,0 | | | | 30,0 | | 8 | | 1,0 | 80 |
| 29 | 15 | 75,0 | | 19,3 | | | | 5,0 | | | 11 | 12 | 0,7 | 55 |
| 30 | 15 | 95,0 | | | 4,6 | | | | | | 11 | 12 | 0,4 | 45 |
| 31 | 13 | 85,0 | | 10 | 4,5 | | | | | | 7 | 3 | 0,5 | 70 |
| 32 | 14 | 97,4 | | | | | | | | | 4 | 4 | 2,6 | 80 |
| 33 | 17 | | | 60 | | | | | 15 | 20 | 1 | 1 | 5,0 | 100 |
| 34 | 17 | | | 45 | | | | | 15 | 20 | 2 | 2 | 20,0 | 100 |

**Patentansprüche**

**Patentansprüche für folgende Vertragsstaaten : BE, CH, DE, FR, GB, GR, IT, LI, NL, SE**

1. Durch Copolymerisation hydrophiler Monomerer herstellbare Hydrogele, dadurch gekennzeichnet, daß bei der Copolymerisation Verbindungen der allgemeinen Formel

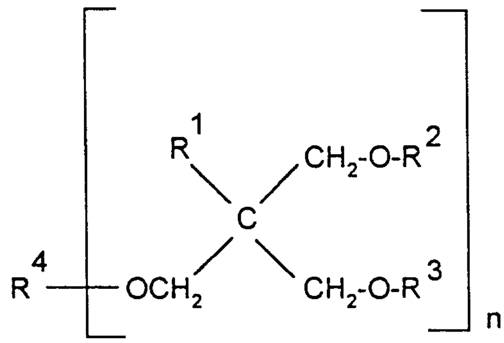

(I)

eingesetzt werden, worin

n 1 oder 2

$R^1$ Alkyl mit 1 bis 4 Kohlenstoffatomen, $CH_2OH$ oder $CH_2OR^7$, worin $R^7$ die Bedeutung von $R^2$ haben kann oder, falls n = 1, zusammen mit $R^4$ eine Gruppe der allgemeinen Formel II

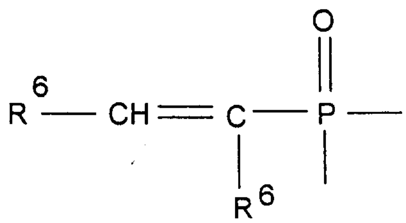

(II)

worin $R^6$ unabhängig voneinander Wasserstoff oder Alkyl mit 1 bis 4 Kohlenstoffatomen bedeutet, bilden kann,

$R^2$ eine Gruppe der allgmeinene Formel III

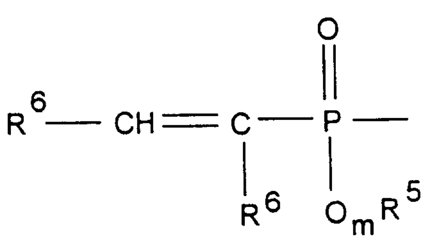

(III)

worin m 0 oder 1 und $R^5$ Alkyl mit 1 bis 4 Kohlenstoffatomen bedeutet, wobei, falls eine Verbindung der allgemeinen Formel I mehr als eine Gruppe der allgemeinen Formel III enthält, die Reste $R^5$ unabhängig voneinander sind,

$R^3$ Wasserstoff oder eine Gruppe der allgemeinen Formel III, wobei $R^2$ und $R^3$ auch zusammen eine Gruppe der allgemeinen Formel II bilden können

$R^4$ für n = 1
Wasserstoff, eine Gruppe der allgemeinen Formel III oder, wenn $R^1$ für Alkyl mit 1 bis 4 Kohlenstoffatomen und $R^2$ und $R^3$ zusammen für eine Gruppe der allgemeinen Formel II stehen, auch eine Gruppe der allgemeinen Formel IV

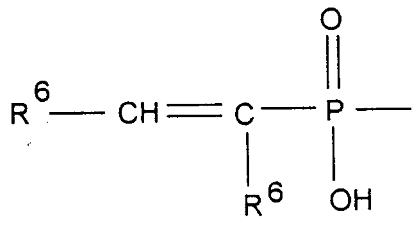

(IV)

und für n = 2
eine Gruppe der allgemeinen Formel II bedeuten.

2. Wasserquellbare Hydrogele gemäß Anspruch 1, dadurch gekennzeichnet, daß bei der Copolymerisation als hydrophile Monomere Acrylsäure, Methacrylsäure, Crotonsäure, 2-Acrylamido-2-methylpropansulfonsäure und -phosphonsäure, Vinylphosphonsäure, Vinylphosphonsäurehalbester, deren Salze, Acrylamid, N-Vinylamide oder Gemische davon eingesetzt werden.

3. Wasserquellbare Hydrogele gemäß Anspruch 1, dadurch gekennzeichnet, daß bei der Copolymerisation als hydrophiles Monomer Acrylsäure und/oder deren Salze eingesetzt werden.

**Patentansprüche für folgenden Vertragsstaat : ES**

1. Verfahren zur Herstellung wasserquellbarer Hydrogele durch Copolymerisation hydrophiler Monomerer, dadurch gekennzeichnet, daß Verbindungen der allgemeinen Formel I

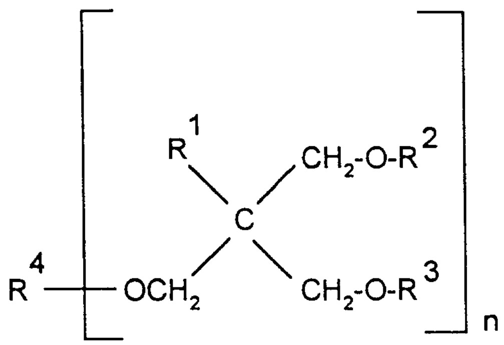

(I)

als Vernetzer eingesetzt werden, worin

n 1 oder 2
$R^1$ Alkyl mit 1 bis 4 Kohlenstoffatomen,
$CH_2OH$ oder $CH_2OR^7$,
worin $R^7$ die Bedeutung von $R^2$ haben kann oder, falls n = 1, zusammen mit $R^4$ eine Gruppe der allgemeinen Formel II

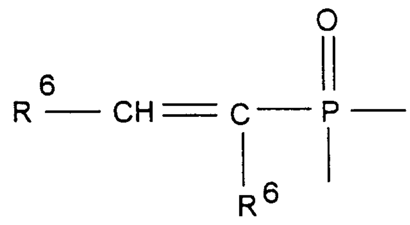

(II)

worin $R^6$ unabhängig voneinander Wasserstoff oder Alkyl mit 1 bis 4 Kohlenstoffatomen bedeutet, bilden kann,

$R^2$ eine Gruppe der allgemeinen Formel III

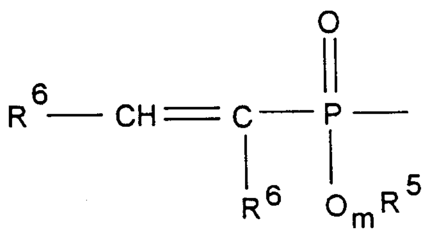

(III)

worin m 0 oder 1 und
$R^5$ Alkyl mit 1 bis 4 Kohlenstoffatomen bedeutet, wobei, falls eine Verbindung der allgemeinen Formel I mehr als eine Gruppe der allgemeinen Formel III enthält, die Reste $R^5$ unabhängig voneinander sind,

$R^3$ Wasserstoff oder eine Gruppe der allgemeinen Formel III, wobei $R^2$ und $R^3$ auch zusammen eine Gruppe der allgemeinen Formel II bilden können,

$R^4$ für n = 1
Wasserstoff, eine Gruppe der allgemeinen Formel III oder, wenn $R^1$ für Alkyl mit 1 bis 4 Kohlenstoffatomen und $R^2$ und R3 zusammen für eine Gruppe der allgemeinen Formel II stehen, auch eine Gruppe der allgemeinen Formel IV

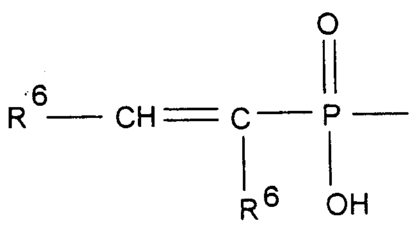

(IV)

und für n = 2
eine Gruppe der allgemeinen Formel II bedeuten.

2. Verfahren gemäß Anspruch 1, dadurch gekennzeichnet, daß als hydrophile Monomere Acrylsäure, Methacrylsäure, Crotonsäure, 2-Acrylamido-2-methylpropansulfonsäure und -phosphonsäure, Vinylphosphonsäure, Vinylphosphonsäurehalbester, deren Salze, Acrylamid, N-Vinylamide oder Gemische davon eingesetzt werden.

3. Verfahren gemäß Anspruch 1, dadurch gekennzeichnet, daß als hydrophiles Monomer Acrylsäure und/oder deren Salze eingesetzt werden.

## Claims

**Claims for the following Contracting States : BE, CH, DE, FR, GB, GR, IT, LI, NL, SE**

1. Hydrogels which can be prepared by copolymerization of hydrophilic monomers, wherein a compound of the formula

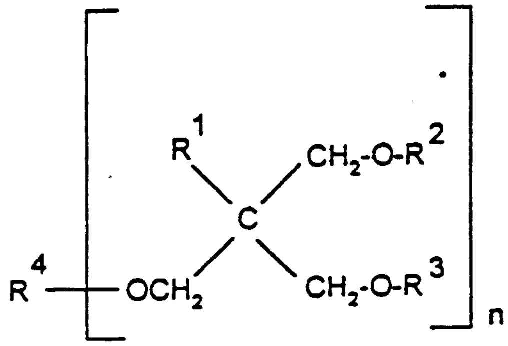

(I)

in which

n is 1 or 2,

$R^1$ is alkyl having 1 to 4 carbon atoms, $CH_2OH$ or $CH_2OR^7$, in which $R^7$ can have the meaning of $R^2$ or, if n = 1, together with $R^4$ can form a group of the formula II

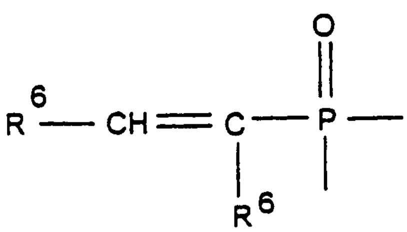

(II)

in which the $R^6$ independently of one another are hydrogen or alkyl having 1 to 4 carbon atoms,

$R^2$ is a group of the formula III

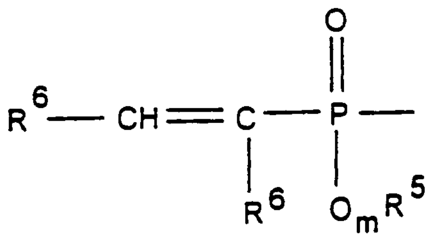

(III)

in which m is 0 or 1 and $R^5$ is alkyl having 1 to 4 carbon atoms, where, if a compound of the formula I contains more than one group of the formula III, the radicals $R^5$ are independent of one another,

$R^3$ is hydrogen or a group of the formula III, in which $R^2$ and $R^3$ together can also form a group of the formula II,

$R^4$ if n = 1,
is hydrogen, a group of the formula III or, if $R^1$ is alkyl having 1 to 4 carbon atoms and $R^2$ and $R^3$ together are a group of the formula II, also a group of the formula IV

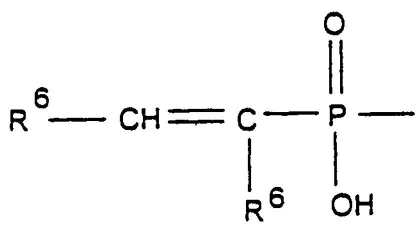

(IV)

and, if n = 2,
is a group of the formula II,

are employed in the copolymerization.

2. Hydrogels as claimed in claim 1, wherein acrylic acid, methacrylic acid, crotonic acid, 2-acrylamido-2-methylpropanesulfonic acid or -phosphonic acid, vinylphosphonic acid, a vinylphosphonic acid half-ester, a salt thereof, acrylamide, an N-vinylamide or a mixture thereof is employed as the hydrophilic monomer in the copolymerization.

3. Hydrogels as claimed in claim 1, wherein acrylic acid and/or a salt thereof is employed as the hydrophilic monomer in the copolymerization.

**Claims for the following Contracting State : ES**

1. A process for the preparation of water-swellable hydrogels by copolymerization of hydrophilic monomers, which comprises employing, as the crosslinking agent, a compound of the formula I

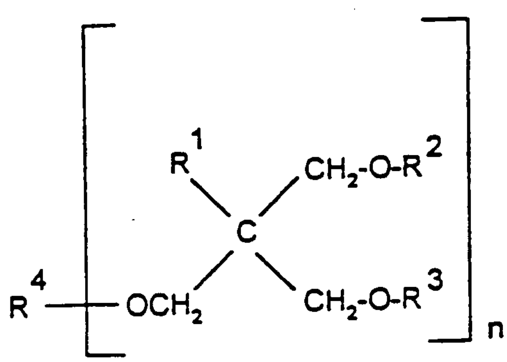

(I)

in which

n is 1 or 2,
$R^1$ is alkyl having 1 to 4 carbon atoms,
$CH_2OH$ or $CH_2OR^7$,
in which $R^7$ can have the meaning of $R^2$ or, if n = 1, together with $R^4$ can form a group of the formula II

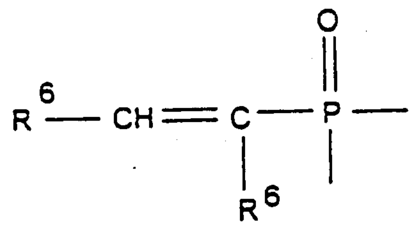

(II)

in which the $R^6$ independently of one another are hydrogen or alkyl having 1 to 4 carbon atoms,

$R^2$ is a group of the formula III

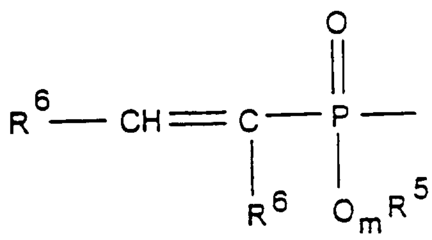

(III)

in which m is 0 or 1 and

$R^5$ is alkyl having 1 to 4 carbon atoms, where, if a compound of the formula I contains more than one group of the formula III, the radicals $R^5$ are independent of one another,

$R^3$ is hydrogen or a group of the formula III, in which $R^2$ and $R^3$ together can also form a group of the formula II,

$R^4$ if n = 1,

is hydrogen, a group of the formula III or, if $R^1$ is alkyl having 1 to 4 carbon atoms and $R^2$ and $R^3$ together are a group of the formula II, also a group of the formula IV

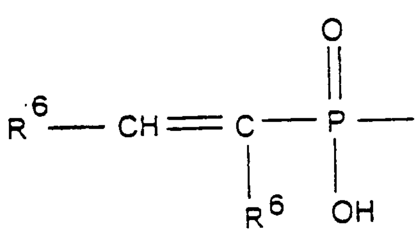

(IV)

and, if n = 2,

is a group of the formula II.

2. The process as claimed in claim 1, wherein acrylic acid, methacrylic acid, crotonic acid, 2-acrylamido-2-methylpropanesulfonic acid or -phosphonic acid, vinylphosphonic acid, a vinylphosphonic acid half-ester, a salt thereof, acrylamide, an N-vinylamide or a mixture thereof is employed as the hydrophilic monomer.

3. The process as claimed in claim 1, wherein acrylic acid and/or a salt thereof is employed as the hydrophilic monomer.

## Revendications

### Revendications pour les Etats contractants suivants : BE, CH, DE, FR, GB, GR, IT, LI, NL, SE

1. Hydrogels qui peuvent être préparés par copolymérisation de monomères hydrophiles, caractérisés en ce que lors de la copolymérisation on utilise des composés de formule générale

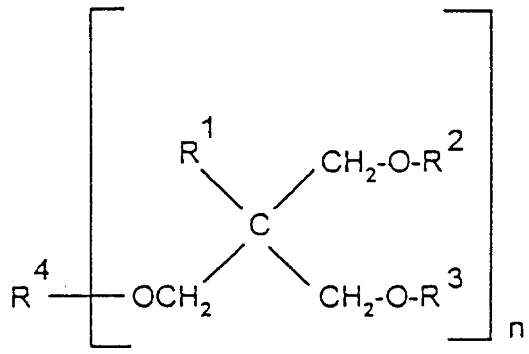

(I)

dans laquelle :

n représente 1 ou 2,

$R^1$ représente alkyle avec 1 à 4 atomes de carbone, $CH_2OH$ ou $CH_2OR^7$, $R^7$ pouvant avoir la signification de $R^2$ ou, si n = 1, pouvant former, ensemble avec $R^4$, un groupe de formule générale II

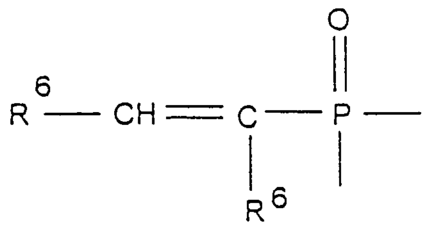

(II)

où les $R^6$, indépendamment l'un de l'autre, représentent l'hydrogène ou alkyle avec 1 à 4 atomes de carbone,

$R^2$ représente un groupe de formule générale III

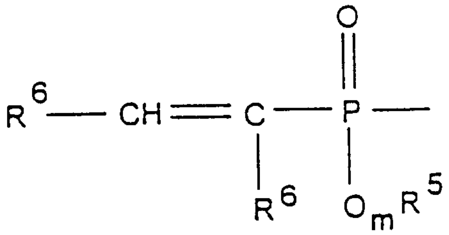

(III)

dans laquelle m représente 0 ou 1 et

$R^5$ représente alkyle avec 1 à 4 atomes de carbone, au cas où un composé de formule générale I contient plus d'un groupe de formule générale III, les radicaux $R^5$ étant indépendants l'un de l'autre, $R^3$ représente l'hydrogène ou un groupe de formule générale III, $R^2$ et $R^3$ pouvant aussi former ensemble un groupe de formule générale II,

$R^4$ pour n = 1

représente l'hydrogène, un groupe de formule générale III ou, si $R^1$ représente alkyle avec 1 à 4 atomes de carbone et $R^2$ et $R^3$ représentent ensemble un groupe de formule générale II, représente aussi un groupe de formule générale IV

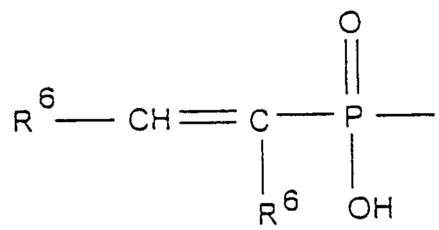

(IV)

et pour n = 2
représente un groupe de formule générale II.

**2.** Hydrogels aptes à gonfler à l'eau selon la revendication 1, caractérisés en ce que lors de la copolymérisation on utilise comme monomères hydrophiles l'acide acrylique, l'acide méthacrylique, l'acide crotonique, les acides 2-acrylamido-2-méthylpropane-sulfonique et -phosphonique, l'acide vinylphosphonique, un semi-ester de l'acide vinylphosphonique, leurs sels, l'acrylamide, des N-vinylamides ou des mélanges de ceux-ci.

**3.** Hydrogels aptes à gonfler à l'eau selon la revendication 1, caractérisés en ce que lors de la copolymérisation on utilise comme monomère hydrophile l'acide acrylique et/ou ses sels.

**Revendications pour l'Etat contractant suivant : ES**

**1.** Procédé de préparation d'hydrogels aptes à gonfler à l'eau par copolymérisation de monomères hydrophiles, caractérisé en ce qu'on utilise comme réticulants des composés de formule générale I

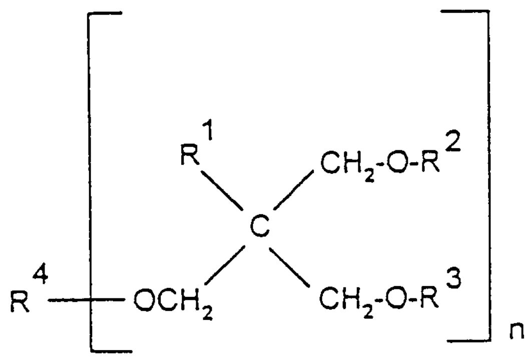

(I)

dans laquelle

n représente 1 ou 2,
$R^1$ représente alkyle avec 1 à 4 atomes de carbone, $CH_2OH$ ou $CH_2OR^7$, $R^7$ pouvant avoir la signification de $R^2$ ou, si n = 1, pouvant former, ensemble avec $R^4$, un groupe de formule générale II

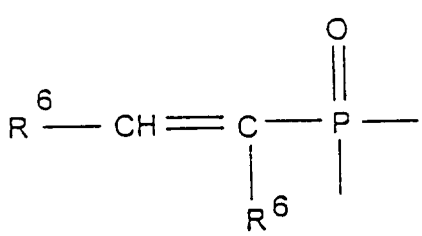

(II)

où les $R^6$, indépendamment l'un de l'autre, représentent l'hydrogène ou alkyle avec 1 à 4 atomes de carbone,
$R^2$ représente un groupe de formule générale III

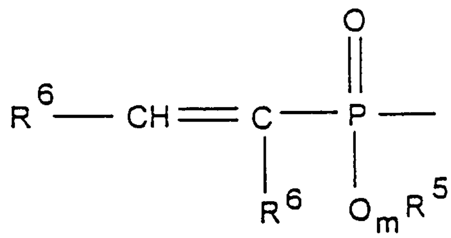

(III)

dans laquelle m représente 0 ou 1 et

$R^5$ représente alkyle avec 1 à 4 atomes de carbone, au cas où un composé de formule générale I contient plus d'un groupe de formule générale III, les radicaux $R^5$ étant indépendants l'un de l'autre, $R^3$ représente l'hydrogène ou un groupe de formule générale III, $R^2$ et $R^3$ pouvant aussi former ensemble un groupe de formule générale II,

$R^4$ pour n = 1

représente l'hydrogène, un groupe de formule générale III ou, si $R^1$ représente alkyle avec 1 à 4 atomes de carbone et $R^2$ et $R^3$ représentent ensemble un groupe de formule générale II, représente aussi un groupe de formule générale IV

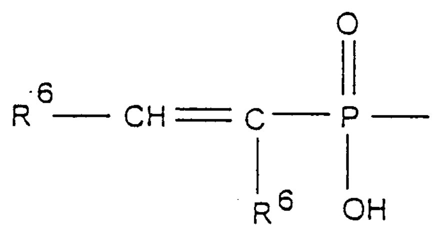

(IV)

et pour n = 2

représente un groupe de formule générale II.

**2.** Procédé selon la revendication 1, caractérisé en ce qu'on utilise comme monomères hydrophiles l'acide acrylique, l'acide méthacrylique, l'acide crotonique, les acides 2-acrylamido-2-méthylpropane-sulfonique et -phosphonique, l'acide vinylphosphonique, un semi-ester de l'acide vinylphosphonique, leurs sels, l'acrylamide, des N-vinylamides ou des mélanges de ceux-ci.

**3.** Procédé selon la revendication 1, caractérisé en ce qu'on utilise comme monomère hydrophile l'acide acrylique et/ou ses sels.